# EUROPÄISCHE PATENTSCHRIFT

(11) **EP 3 000 404 B1**
(45) Veröffentlichungstag und Bekanntmachung des Hinweises auf die Patenterteilung: **06.06.2018**
(21) Anmeldenummer: 15186390.9
(22) Anmeldetag: 23.09.2015
(51) Int. Cl.: A61B 17/42, A61B 17/00

(54) **MEDIZINISCHES INSTRUMENT UND KOPPLUNGSEINRICHTUNG ZUR VERBINDUNG ZWEIER BESTANDTEILE EINES MEDIZINISCHEN INSTRUMENTS**
MEDICAL INSTRUMENT AND COUPLING DEVICE FOR CONNECTING TWO COMPONENTS OF A MEDICAL INSTRUMENT
INSTRUMENT MEDICAL ET DISPOSITIF DE LIAISON DESTINE A RELIER DEUX COMPOSANTS D'UN INSTRUMENT MEDICAL

(30) Priorität: 23.09.2014 DE 102014113751
(43) Veröffentlichungstag der Anmeldung: 30.03.2016
(73) Patentinhaber: Karl Storz SE & Co. KG, 78532 Tuttlingen (DE)
(72) Erfinder: Hermle, Rainer, 78532 Tuttlingen (DE); Egle, Michael, 78532 Tuttlingen (DE); Kramer, Claus, 78532 Tuttlingen (DE)

(56) Entgegenhaltungen:
- DE-A1- 10 257 760
- DE-C1- 19 851 268
- US-A- 5 466 020
- US-A1- 2013 066 328

## Beschreibung

Die vorliegende Erfindung ist auf ein medizinisches Instrument, insbesondere einen Uterus-Manipulator, und eine Einrichtung zur Verbindung zweier Bestandteile eines solchen medizinischen Instruments bezogen, wobei die beiden Bestandteile des medizinischen Instruments relativ zu einander verschiebbar sind.

Zahlreiche medizinische Instrumente, insbesondere medizinische Instrumente für mikroinvasive Maßnahmen sind zerlegbar, um nach jeder Verwendung eine vollständige Reinigung aller Oberflächen zu ermöglichen. Bei einigen medizinischen Instrumenten sollen zwei oder mehr Bestandteile im mechanisch verbundenen Zustand relativ zueinander bewegt werden können. Beides gleichzeitig ist beispielsweise bei Uterus-Manipulatoren nicht oder nicht befriedigend realisierbar.

Hierzu schlägt US 5466020 A für geeignete Zerlegbarkeit von Bestandteilen eines medizinischen Instruments eine **Kopplungseinrichtung** mit zwei Bauteilen vor, die zwei Bestandteile eines medizinischen Instruments mechanischen verbinden lässt, wobei die beiden Bauteile über eine Renkverbindung verbindbar sind, und im verbundenen Zustand eine Rotation der beiden Bauteile relativ zueinander um eine Rotationsachse gewährleistet ist. Ferner ermöglicht in US 5466020 A eine **Rasteinrichtung,** dass die beiden Bauteile über eine Rasteinrichtung in zwei bestimmten Winkelpositionen relativ einander zu arretiert werden, was über Rastnasen und korrespondierende Ausnehmungen ermöglicht ist.

DE 10257760 A1 zeigt wiederum zum Zerlegen eines medizinischen Instruments eine Kopplungseinrichtung mit zwei Bauteilen zum mechanischen Verbinden zwei Bestandteile des Instruments Auch hier wirkt eine Renkverbindung, die im verbundenen Zustand eine Rotation der beiden Bauteile gegeneinander um eine Rotationsachse erlaubt, wobei auch eine Klemmung der beiden Bauteile in zwei bestimmten Winkelpositionen relativ einander vorgesehen ist.

US 2013/066328 A1 offenbart ein medizinisches Schaft Instrument zur Behandlung eines menschlichen oder tierischen Körpers, wobei das Instrument eine Kopplungseinrichtung mit mehreren vorbestimmten Konfigurationen, zur mechanischen Verbindung der Wirkeinrichtung mit dem Schaft umfasst, und die Konfigurationen einen zusammensetzten und einen getrennten Zustand von Wirkeinrichtung und Schaft ermöglicht, wobei im getrennten Zustand die Wirkeinrichtung entlang dem Schaft zwischen zwei proximalen bzw. distalen Positionen verschiebbar ist.

Eine Aufgabe der vorliegenden Erfindung besteht darin, eine verbesserte Kopplungseinrichtung zur mechanischen Verbindung von Bestandteilen eines medizinischen Instruments und ein verbessertes medizinisches Instrument zu schaffen.

Diese Aufgabe wird durch den Gegenstand des unabhängigen Anspruchs gelöst. Weiterbildungen sind in den abhängigen Ansprüchen angegeben.

Ausführungsbeispiele der vorliegenden Erfindung beruhen auf der Idee, eine Kopplungseinrichtung aus zwei Bauteilen zu bilden, die durch eine Renkverbindung, insbesondere eine Bajonettverbindung, derart miteinander verbindbar sind, dass sie in mehreren relativen räumlichen Konfigurationen, insbesondere in mehreren relativen Winkelpositionen miteinander verbunden sind. Zum lösbaren Halten der beiden Bauteile in jeder von mehreren vorbestimmten Konfigurationen, insbesondere Winkelpositionen, ist insbesondere eine Rasteinrichtung vorgesehen.

Eine derartige Kopplungseinrichtung kann beispielsweise bei einer ersten Konfiguration ein Zusammensetzen und ein zerstörungsfreies Trennen von Bestandteilen eines medizinischen Instruments ermöglichen, bei einer zweiten Konfiguration Bestandteile des medizinischen Instruments derart miteinander verbinden, dass sie relativ zueinander innerhalb eines vorbestimmten Bereichs bewegbar sind, jedoch nicht von einander getrennt werden können, und bei einer dritten Konfiguration Bestandteile des medizinischen Instruments in einer vorbestimmten oder in einer von mehreren alternativen vorbestimmten oder beliebigen relativen Positionen zu halten bzw. zu arretieren.

Eine derartige Kopplungseinrichtung kann insbesondere eine besonders einfache Konstruktion eines Uterus-Manipulators, bei dem die Glocke vom Schaft abnehmbar und im verbundenen Zustand entlang des Schafts zwischen zwei Endpositionen verschiebbar und optional in mindestens einer Position arretierbar ist, ermöglichen.

Eine Kopplungseinrichtung zur mechanischen Verbindung eines ersten Bestandteils und eines zweiten Bestandteils eines medizinischen Instruments umfasst ein erstes Bauteil, ein zweites Bauteil, wobei das erste Bauteil und das zweite Bauteil ausgebildet sind, um durch eine Renkverbindung, die im verbundenen Zustand eine Rotation des zweiten Bauteils relativ zum ersten Bauteil um eine Rotationsachse ermöglicht, miteinander verbunden zu werden, und eine Rasteinrichtung zum alternativen Arretieren des zweiten Bauteils in einer ersten vorbestimmten Winkelposition oder in einer zweiten vorbestimmten Winkelposition relativ zum ersten Bauteil.

Die Kopplungseinrichtung ist insbesondere zur mechanischen Verbindung von Bestandteilen eines mikroinvasiven medizinischen Instruments, beispielsweise eines Rohrschaftinstruments, vorgesehen und ausgebildet. Als Rohrschaftinstrument wird jedes medizinische Instrument bezeichnet, das einen geraden, gekrümmten oder krümmbaren, zumindest abschnittsweise starren oder zumindest abschnittsweise flexiblen Schaft umfasst, wobei der Schaft insbesondere mindestens ein starres oder flexibles Rohr aufweist. Als Rohrschaftinstrumente werden unter anderen Uterus-Manipulatoren und Endoskope bezeichnet. Die Kopplungseinrichtung kann zur mechanischen Verbindung von Bestandteilen, von denen mindestens eines selbst ein medizinisches Instrument darstellt, zu einem komplexeren medizinischen Instrument ausgebildet sein.

Eine Renkverbindung ist eine Verbindung, die hergestellt wird, indem zwei Bauteile zunächst mittels einer insbesondere zumindest näherungsweise linearen Bewegung aufeinander zu bewegt und dann relativ zueinander um eine Achse rotiert werden. Dabei ist die Achse, um die insbesondere das zweite Bauteil relativ zum ersten Bauteil rotiert wird, um die mechanische Verbindung beider Bauteile herzustellen, insbesondere parallel zur Richtung der vorangehenden linearen Relativbewegung. Die Rotationsachse der durch die Renkverbindung ermöglichten Rotation der miteinander verbundenen Bauteile relativ zueinander, ist insbesondere identisch oder parallel zu der Achse, um die das zweite Bauteil relativ zum ersten Bauteil rotiert wird, um die Renkverbindung zu bilden.

Die Trennbarkeit der beiden Bauteile der Kopplungseinrichtung an der Renkverbindung ist nicht zu verwechseln mit der Trennbarkeit der Bestandteile des medizinischen Instruments. Vielmehr kann die Kopplungseinrichtung in einer ihrer Konfigurationen bzw. bei einer vorbestimmten Winkelposition des zweiten Bauteils relativ zum ersten Bauteil eine mechanische Verbindung von Bestandteilen eines medizinischen Instruments und in einer anderen Konfiguration bzw. bei einer anderen Winkelposition des zweiten Bauteils relativ zu dem mit ihm verbundenen ersten Bauteil eine zerstörungsfreie Trennung von Bestandteilen des medizinischen Instruments ermöglichen. Dabei sind in beiden Konfigurationen der Kopplungseinrichtung deren Bauteile durch die Renkverbindung miteinander verbunden, auch bei der Konfiguration, bei der die Bestandteile des medizinischen Instruments zerstörungsfrei von einander getrennt werden können.

Die Rasteinrichtung ist insbesondere ausgebildet, um das zweite Bauteil alternativ in einer von mehreren alternativen und voneinander verschiedenen vorbestimmten Winkelpositionen lösbar zu arretieren. Alternativ kann die Rasteinrichtung ausgebildet sein, um das zweite Bauteil zumindest in einer vorbestimmten Winkelposition relativ zum ersten Bauteil unlösbar bzw. nicht zerstörungsfrei lösbar zu arretieren.

Die insbesondere durch die Rasteinrichtung allein definierten verschiedenen vorbestimmten Konfigurationen bzw. Winkelpositionen des zweiten Bauteils relativ zum ersten Bauteil können mehreren verschiedenen Wirkungen der Kopplungseinrichtung, die durch die verschiedenen Winkelpositionen bedingt sind, entsprechen.

Die Kopplungseinrichtung kann insbesondere die Reinigung vereinfachen und das Austauschen oder Ersetzen von Bestandteilen im Fall eines Schadens oder Defekts oder zur Anpassung an die spezifischen Erfordernisse einer bestimmen medizinischen Maßnahme bei einem bestimmten Patienten ermöglichen. Durch die Renkverbindung zwischen den Bauteilen der Kopplungseinrichtung ist überdies die Kopplungseinrichtung selbst leicht zerlegbar und kann deshalb weitgehend oder vollständig gereinigt werden.

Bei einer Kopplungseinrichtung, wie sie hier beschrieben ist, ist die Kopplungseinrichtung vorgesehen und ausgebildet, um bei der ersten Winkelposition ein Zusammensetzen und ein zerstörungsfreies Trennen des ersten Bestandteils und des zweiten Bestandteils des medizinischen Instruments zu ermöglichen, und um bei der zweiten Winkelposition eine mechanische Verbindung des ersten Bestandteils und des zweiten Bestandteils des medizinischen Instruments zu schaffen.

Insbesondere ist die Kopplungseinrichtung vorgesehen und ausgebildet, um bei der ersten Winkelposition zusammen mit dem zweiten Bestandteil des medizinischen Instruments auf einen Schaft als ersten Bestandteil des medizinischen Instruments aufgeschoben zu werden. Damit schafft die Kopplungseinrichtung beispielsweise die Möglichkeit, eine Glocke oder eine andere Wirkeinrichtung auf den Schaft eines Uterus-Manipulators aufzuschieben, mit diesem zu verbinden und von ihm wieder zerstörungsfrei zu trennen.

Eine Kopplungseinrichtung, wie sie hier beschrieben ist, ist insbesondere vorgesehen und ausgebildet, um bei der zweiten Winkelposition den ersten Bestandteil und den zweiten Bestandteil des medizinischen Instruments derart zu verbinden, dass der zweite Bestandteil relativ zu dem ersten Bestandteil zwischen einer ersten vorbestimmten Endposition und einer zweiten vorbestimmten Endposition bewegbar ist.

Die Kopplungseinrichtung ist insbesondere vorgesehen und ausgebildet, um bei der zweiten Winkelposition zusammen mit der Wirkeinrichtung linear, beispielsweise entlang eines geraden oder gekrümmten Schafts, zwischen einer proximalen Endposition und einer distalen Endposition bewegbar zu sein.

Bei einer Kopplungseinrichtung, wie sie hier beschrieben ist, ist die Rasteinrichtung insbesondere ferner vorgesehen und ausgebildet, um das zweite Bauteil relativ zum ersten Bauteil alternativ in einer dritten vorbestimmten Winkelposition zu arretieren, wobei die Kopplungseinrichtung vorgesehen und ausgebildet ist, um bei der dritten Winkelposition eine im Wesentlichen starre Verbindung des ersten Bestandteils und des zweiten Bestandteils des medizinischen Instruments zu schaffen.

Bestandteile eines medizinischen Instruments sind im Wesentlichen starr verbunden, wenn sie von unvermeidbarem oder nicht mit vernünftigem Aufwand vermeidbarem Spiel oder von Spiel, das im Sinne angemessener Fertigungskosten oder geringer Reibung in Kauf genommen wird oder gewünscht ist, abgesehen nicht mehr relativ zueinander bewegt, insbesondere relativ zueinander verschoben werden können.

Bei einer Kopplungseinrichtung, wie sie hier beschrieben ist, umfasst die Rasteinrichtung eine Rastnase und mehrere zur Rastnase korrespondierende Ausnehmungen, die die erste Winkelposition und die zweite Winkelposition vorbestimmen, oder eine Ausnehmung und mehrere zur Ausnehmung korrespondierende Rastnasen, die die erste Winkelposition und die zweite Winkelposition vorbestimmen.

Die Rasteinrichtung kann mehrere Rastnasen und mehrere Ausnehmungen bzw. Vertiefungen aufweisen, um das zweite Bauteil relativ zum ersten Bauteil in allen Winkelpositionen zu arretieren, bei denen mindestens eine Rastnase in eine gegenüberliegende und korrespondierende Ausnehmung eingreift. Die Rastnase oder die Rastnasen sind insbesondere jeweils selbst elastisch oder an einer elastischen Einrichtung angeordnet, um gegen eine elastische Kraft ausgelenkt und aus der Ausnehmung heraus bewegt werden zu können.

Insbesondere sind die Ausnehmung oder die Ausnehmungen am ersten Bauteil (beispielsweise mit dem ersten Bauteil starr oder elastisch verbunden) und die Rastnase oder die Rastnasen am zweiten Bauteil (beispielsweise mit dem zweiten Bauteil starr oder elastisch verbunden) vorgesehen. Alternativ oder zusätzlich sind eine oder mehrere Rastnasen am ersten Bauteil und eine oder mehrere Ausnehmungen am zweiten Bauteil vorgesehen.

Bei einer Kopplungseinrichtung, wie sie hier beschrieben ist, ist zumindest entweder eine Rastnase oder eine Ausnehmung der Rasteinrichtung in einer Richtung, die im Wesentlichen parallel zur Rotationsachse ist, elastisch auslenkbar. Eine Richtung ist im Wesentlichen parallel zur Rotationsachse, um die das zweite Bauteil relativ zum ersten Bauteil rotierbar ist, wenn die Richtung und die Rotationsachse einen Winkel einschließen, der nicht größer als 45 Grad oder nicht größer als 30 Grad oder nicht größer als 20 Grad oder nicht größer als 10 Grad oder nicht größer als 5 Grad ist.

Bei einer Kopplungseinrichtung, wie sie hier beschrieben ist, ist zumindest entweder eine Rastnase oder eine Ausnehmung der Rasteinrichtung in einer Richtung, die im Wesentlichen orthogonal zur Rotationsache ist, elastisch auslenkbar.

Eine Richtung ist im Wesentlichen orthogonal zur Rotationsachse, um die das zweite Bauteil relativ zum ersten Bauteil rotierbar ist, wenn der Winkel zwischen der Richtung und der Rotationsachse im Bereich von 45 Grad bis 90 Grad oder im Bereich von 60 Grad bis 90 Grad oder im Bereich von 70 Grad bis 90 Grad oder im Bereich von 80 Grad bis 90 Grad oder im Bereich von 85 Grad bis 90 Grad liegt.

Bei einer Kopplungseinrichtung, wie sie hier beschrieben ist, ist eine Rastnase der Rasteinrichtung insbesondere an einer einseitig oder beidseitig befestigten streifenförmigen, elastischen Einrichtung angeordnet.

Die Rastnase ist insbesondere am freien Ende einer einseitig befestigten bzw. eingespannten streifenförmigen, elastischen Einrichtung angeordnet. Alternativ kann die Rastnase zwischen den Enden, insbesondere in der Mitte zwischen den Enden einer beidseitig befestigen bzw. eingespannten streifenförmigen, elastischen Einrichtung angeordnet. Alternativ können mehrere Rastnasen der Rasteinrichtung oder eine oder mehrere Ausnehmungen an der streifenförmigen elastischen Einrichtung angeordnet sein. Die streifenförmige elastische Einrichtung bildet insbesondere einen - miniaturisierten - einseitig oder beidseitig eingespannten elastischen Balken. Die streifenförmige elastische Einrichtung kann durch einen L-förmigen Einschnitt (insbesondere für eine einseitige Befestigung) oder durch einen geraden Schnitt (insbesondere im Falle einer beidseitigen Befestigung) an einem Rand des ersten Bauteils oder des zweiten Bauteils gebildet sein.

Bei einer Kopplungseinrichtung, wie sie hier beschrieben ist, ist die elastische Einrichtung insbesondere einstückig mit dem ersten Bauteil oder einstückig mit dem zweiten Bauteil ausgebildet.

Bei einer Kopplungseinrichtung, wie sie hier beschrieben ist, sind das erste Bauteil und das zweite Bauteil insbesondere jeweils im Wesentlichen rohrförmig, wobei das erste Bauteil zumindest teilweise in dem zweiten Bauteil angeordnet ist, wenn das zweite Bauteil mit dem ersten Bauteil durch die Renkverbindung verbunden ist.

Bei einer Kopplungseinrichtung, wie sie hier beschrieben ist, sind das erste Bauteil und das zweite Bauteil insbesondere jeweils im Wesentlichen rohrförmig, wobei das zweite Bauteil zumindest teilweise in dem ersten Bauteil angeordnet ist, wenn das zweite Bauteil mit dem ersten Bauteil durch die Renkverbindung verbunden ist.

Das erste Bauteil und das zweite Bauteil sind also insbesondere koaxial angeordnet, wenn sie durch die Renkverbindung mechanisch verbunden sind. Das erste Bauteil und das zweite Bauteil weisen insbesondere jeweils zumindest abschnittsweise die Gestalt der Mantelfläche eines Kreiszylinders oder eines geraden Kegelstumpfs mit kreisförmiger Grundfläche auf.

Eine Kopplungseinrichtung, wie sie hier beschrieben ist, umschließt insbesondere einen im Wesentlichen zylindrischen Innenraum, wobei das zweite Bauteil einen Bereich aufweist, der zumindest in einer Winkelposition in den zylindrischen Innenraum ragt.

Eine Kopplungseinrichtung, wie sie hier beschrieben ist, umschließt insbesondere einen im Wesentlichen kreiszylindrischen Innenraum, wobei das erste Bauteil einen in den kreiszylindrischen Innenraum ragenden Bereich aufweist und das zweite Bauteil einen in den kreiszylindrischen Innenraum ragenden Bereich aufweist.

Die Kopplungseinrichtung ist insbesondere zur Kopplung mit einem kreiszylindrischen Schaft, der eine oder mehrere Nuten oder Abflachungen aufweist, vorgesehen. Die Kopplungseinrichtung kann dann beispielsweise parallel zur Längsachse des Schafts verschoben werden, wenn beide nach innen ragende Bereiche an den beiden Bauteilen in die gleichen oder zwei verschiedene Nuten oder Abflachungen eingreifen. An Stellen am Schaft, an denen dieser eine in umfänglicher Richtung sich erstreckende Nut aufweist, kann beispielsweise das zweite Bauteil relativ zum ersten Bauteil rotiert werden, während der nach innen ragende Bereich des zweiten Bauteils in die in umfänglicher Richtung sich erstreckende Nut eingreift. Dies kann einer Verriegelung der Kopplungseinrichtung am Ort der in umfänglicher Richtung sich erstreckenden Nut oder einer Überführung des nach innen ragenden Bereichs am zweiten Bauteil in eine andere Nut oder Abflachung parallel zur Längsachse des Schafts ermöglichen.

Bei einer Kopplungseinrichtung, wie sie hier beschrieben ist, weist das erste Bauteil insbesondere einen Kragen auf, wobei die Rasteinrichtung an dem Kragen am ersten Bauteil und an einem dem Kragen gegenüberliegenden Rand des zweiten Bauteils angeordnet ist.

Insbesondere sind die Ausnehmung oder die Ausnehmungen der Rasteinrichtung am Kragen am ersten Bauteil und die Rastnase oder die Rastnasen an dem dem Kragen am ersten Bauteil gegenüberliegenden Rand des zweiten Bauteils angeordnet. Alternativ oder zusätzlich können eine oder mehrere Rastnasen der Rasteinrichtung am Kragen am ersten Bauteil und eine oder mehrere Ausnehmungen der Rasteinrichtung an dem dem Kragen am ersten Bauteil gegenüberliegenden Rand des zweiten Bauteils angeordnet sein.

Insbesondere öffnen sich die Ausnehmung oder die Ausnehmungen in axialer Richtung, und die Rastnase oder die Rastnasen greifen in axialer Richtung in die Ausnehmung oder in die Ausnehmungen ein. Das Maß, um das der Kragen am ersten Bauteil übersteht, entspricht insbesondere der Wandstärke des zweiten Bauteils zuzüglich des Spiels zwischen der Innenwand des zweiten Bauteils und der Außenwand des ersten Bauteils.

Wenn das erste Bauteil im Wesentlichen innerhalb des zweiten Bauteils angeordnet ist, kann der Kragen nach außen ragen. Wenn das zweite Bauteil im Wesentlichen innerhalb des ersten Bauteils angeordnet ist, kann ein Kragen mit entsprechenden Merkmalen und Eigenschaften nach innen ragen. In beiden Fällen kann alternativ oder zusätzlich zu einem Kragen am ersten Bauteil ein in entgegengesetzter Richtung ragender Kragen mit entsprechenden Merkmalen am zweiten Bauteil vorgesehen sein.

Ein medizinisches Instrument umfasst einen ersten Bestandteil, einen zweiten Bestandteil und eine Kopplungseinrichtung, wie sie hier beschrieben ist, zur mechanischen Verbindung des ersten Bestandteils und des zweiten Bestandteils des medizinischen Instruments.

Eines der beiden Bauteile der Kopplungseinrichtung, insbesondere das erste Bauteil der Kopplungseinrichtung, kann starr und unlösbar mit einem der beiden Bestandteile des medizinischen Instruments verbunden, insbesondere mit diesem Bestandteil einstückig hergestellt oder nach separater Herstellung stoff-, kraft- und/oder formschlüssig gefügt sein.

Ein medizinisches Instruments umfasst einen Schaft, eine Wirkeinrichtung zur Einwirkung auf einen Teil eines menschlichen oder tierischen Körpers und eine Kopplungseinrichtung mit mehreren vorbestimmten Konfigurationen zur mechanischen Verbindung der Wirkeinrichtung mit dem Schaft, wobei bei einer ersten Konfiguration der Kopplungseinrichtung ein Zusammensetzen und eine zerstörungsfreie Trennung von Wirkeinrichtung und Schaft möglich sind, und wobei bei einer zweiten Konfiguration der Kopplungseinrichtung die Wirkeinrichtung mit dem Schaft derart mechanisch verbunden ist, dass die Wirkeinrichtung entlang dem Schaft zwischen einer proximalen Position und einer distalen Position verschiebbar ist.

Der Schaft weist insbesondere eine oder mehrere Nuten oder Abflachungen, die sich in Längsrichtung des Schafts erstrecken, und eine oder mehrere in umfänglicher Richtung sich erstreckende Nuten auf, wobei die Kopplungseinrichtung die beschriebenen nach innen ragende Bereiche am ersten Bauteil und am zweiten Bauteil aufweist, die in die Nuten und/oder Abflachungen eingreifen.

Ein medizinisches Instrument, wie es hier beschrieben ist, ist insbesondere für mikroinvasive medizinische Maßnahmen vorgesehen und ausgebildet. Ein medizinisches Instrument, wie es hier beschrieben ist, ist insbesondere ein medizinisches Rohrschaftinstrument, beispielsweise ein Uterus-Manipulator.

Die Wirkeinrichtung ist insbesondere zur mechanischen und/oder zur elektrochirurgischen Einwirkung auf ein Organ oder Gewebe eines menschlichen oder tierischen Körpers vorgesehen, beispielsweise zum Halten, Abtrennen oder Veröden. Die Wirkeinrichtung umfasst beispielsweise eine Glocke eines Uterus-Manipulators.

Bei einem medizinischen Instrument, wie es hier beschrieben ist, ist insbesondere bei einer dritten Konfiguration der Kopplungseinrichtung die Wirkeinrichtung in einer Position am Schaft arretiert.

Bei der dritten Konfiguration der Kopplungseinrichtung ist die Wirkeinrichtung insbesondere in einer vorbestimmten Position oder einer von mehreren vorbestimmten Positionen am Schaft arretiert. Die Arretierung erfolgt beispielsweise indem der nach innen ragende Bereich am ersten Bauteil in eine in Längsrichtung des Schafts sich erstreckende Nut oder Ausnehmung eingreift und der nach innen ragende Bereich des zweiten Bauteils in eine in umfänglicher Richtung sich erstreckende Nut am Schaft eingreift oder umgekehrt.

Bei einem medizinischen Instrument, wie es hier beschrieben ist, umfasst die Kopplungseinrichtung insbesondere ein erstes Bauteil, das mit der Wirkeinrichtung verbunden ist, und ein zweites Bauteil, das relativ zu dem ersten Bauteil um die Längsachse des Schafts rotierbar ist, und das bei verschiedenen Konfigurationen der Kopplungseinrichtung unterschiedliche Winkelpositionen relativ zum ersten Bauteil einnimmt.

Bei einem medizinischen Instrument, wie es hier beschrieben ist, ist die Kopplungseinrichtung insbesondere eine Kopplungseinrichtung, wie sie hier beschrieben ist.

Bei einem medizinischen Instrument, wie es hier beschrieben ist, umfasst die Kopplungseinrichtung insbesondere eine Spannzange oder eine andere Einrichtung zur kraftschlüssigen Verbindung des ersten Bestandteils mit dem zweiten Bestandteil des Instruments.

Die Spannzange ist insbesondere ausgebildet, um durch axiale Verschiebung und/oder Rotation eines Bauteils relativ zu einem anderen Bauteil eine oder mehrere Keile, Backen oder andere zumindest auch in radialer Richtung bewegbare Einrichtungen nach radial innen zu verschieben, um eine kraft- bzw. reibschlüssige Verbindung zu bilden.

Ein medizinisches Instrument, wie es hier beschrieben ist, ist insbesondere ein Uterus-Manipulator.

### Kurzbeschreibung der Figuren

Nachfolgend werden Ausführungsformen anhand der beigefügten Figuren näher erläutert. Es zeigen:
- Figur 1: eine schematische axonometrische Darstellung eines Uterus-Manipulators;
- Figur 2: eine weitere schematische axonometrische Darstellung des Uterus-Manipulators aus Figur 1;
- Figur 3: eine weitere schematische Darstellung des Uterus-Manipulators aus den Figuren 1 und 2;
- Figur 4: eine weitere schematische Darstellung des Uterus-Manipulators aus den Figuren 1 bis 3;
- Figur 5: eine schematische Darstellung einer Kopplungseinrichtung des Uterus-Manipulators aus den Figuren 1 bis 4;
- Figur 6: eine weitere schematische Darstellung der Kopplungseinrichtung aus Figur 5;
- Figur 7: eine schematische Darstellung eines Längsschnitts durch die Kopplungseinrichtung aus den Figuren 5 und 6;
- Figur 8: eine weitere schematische Darstellung eines Längsschnitts durch die Kopplungseinrichtung aus den Figuren 5 bis 7;
- Figur 9: eine schematische Darstellung eines Querschnitts durch den Schaft des UterusManipulators aus den Figuren 1 bis 4 mit der Kopplungseinrichtung aus den Figuren 5 bis 8;
- Figur 10: eine weitere schematische Darstellung eines Querschnitts durch den Schaft des UterusManipulators aus den Figuren 1 bis 4 mit der Kopplungseinrichtung aus den Figuren 5 bis 8;
- Figur 11: eine weitere schematische Darstellung eines Querschnitts durch den Schaft des UterusManipulators aus den Figuren 1 bis 4 mit der Kopplungseinrichtung aus den Figuren 5 bis 8;
- Figur 12: eine schematische axonometrische Darstellung einer alternativen Ausführungsform eines Teils einer Kopplungseinrichtung;
- Figur 13: eine schematische axonometrische Darstellung einer weiteren alternativen Ausführungsform einer Kopplungseinrichtung;
- Figur 14: eine schematische Darstellung eines Längsschnitts durch die Kopplungseinrichtung aus Figur 13;
- Figur 15: ein schematisches Flussdiagramm eines Verfahrens zum Vorbereiten einer Verwendung eines medizinischen Instruments.

### Beschreibung der Ausführungsformen

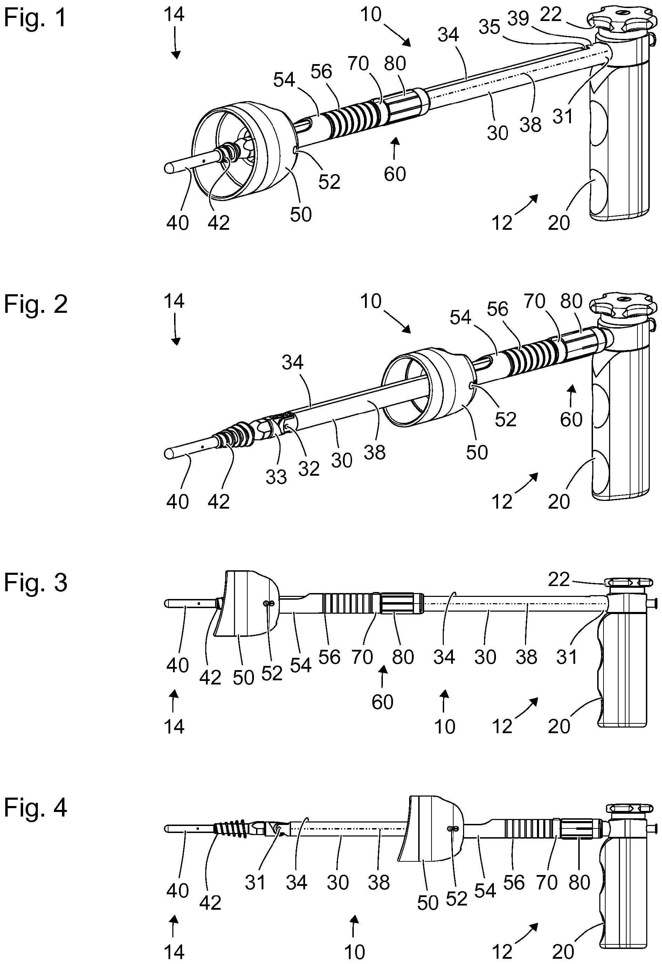

Figur 1 zeigt eine schematische Darstellung eines Uterus-Manipulators 10 mit einem proximalen Ende 12 und einem distalen Ende 14. Am proximalen Ende 12 des Uterus-Manipulators 10 ist ein rotierbarer Handgriff 20 mit einem Drehrad 22 zur Arretierung vorgesehen. Der Uterus-Manipulator 10 umfasst einen Schaft 30 mit einem proximalen Ende 31, das mit dem Handgriff 20 verbunden ist, einem in Figur 1 nicht sichtbaren Gelenk und einem in Figur 1 ebenfalls nicht sichtbaren distalen Ende nahe dem distalen Ende 14 des Uterus-Manipulators 10. Der Schaft 30 weist im Wesentlichen die Gestalt eines Kreiszylinders auf.

Der Schaft 30 weist - abweichend von der idealen Gestalt eines Kreiszylinders - eine erste, sich in Längsrichtung des Schafts 30 erstreckende Abflachung 34, eine erste, sich in Richtung des Umfangs des Schafts 30 erstreckende Nut 35 nahe dem proximalen Ende 31 und weitere, in Figur 1 nicht sichtbare Abflachungen und Nuten auf. Die Abflachung 34 verläuft gerade und parallel zur Längsachse 38 des Schafts 30. Im Bereich der Abflachung 34 ist die äußere Kontur des Querschnitts des Schafts 30 nicht kreisbogenförmig gekrümmt, sondern gerade. Am proximalen Ende 31 des Schafts 30 ist ferner eine Ausnehmung 39 vorgesehen.

Am distalen Ende des Schafts 30 ist ein Arbeitseinsatz 40 mit einem Gewinde 42 zur formschlüssigen Verbindung mit dem Uterus vorgesehen. Der Arbeitseinsatz 40 ist mit dem distalen Ende des Schafts 30 insbesondere lösbar verbunden, beispielsweise durch eine Schraub- oder RenkVerbindung. Der Arbeitseinsatz 40 ist insbesondere gegen andere Arbeitseinsätze mit gleichen oder unterschiedlichen Merkmalen, Abmessungen und sonstigen Eigenschaften austauschbar. Dies vereinfacht nicht nur die Reinigung und Sterilisierung, sondern ermöglicht auch die Verwendung des Uterus-Manipulators 10 mit unterschiedlichen Arbeitseinsätzen für unterschiedliche medizinische Maßnahmen.

Der Uterus-Manipulator 10 umfasst ferner eine Glocke 50 aus Keramik oder einem anderen Material, die über ein Gelenk 52 mit einem Rohr 54 verbunden ist. Das Rohr 54 weist einen inneren Querschnitt auf, der so auf den äußeren Querschnitt des Schafts 30 abgestimmt ist, dass das Rohr 54 zusammen mit der Glocke 50 spiel- und reibungsarm entlang des Schafts 30 parallel zu dessen Längsachse 38 verschiebbar ist. An der äußeren Oberfläche des Rohrs 54 ist eine Riffelung 56 in Form umlaufender Nuten vorgesehen, um ein sicheres Halten des Rohrs 54 zu ermöglichen. Bei der dargestellten distalen Position der Glocke 50 und des Rohrs 54 fallen die Schwenkachsen des in Figur 1 nicht sichtbaren Gelenks nahe dem distalen Ende 33 des Schafts 30 und des Gelenks 52 zwischen 50 und Rohr 54 zusammen.

Am proximalen Ende des Rohrs 56 ist eine Kopplungseinrichtung 60 mit einem ersten Bauteil 70 und einem zweiten Bauteil 80 vorgesehen. Das zweite Bauteil 80 ist relativ zum ersten Bauteil 70 zwischen verschiedenen, insbesondere durch Rastungen vorbestimmten Positionen bewegbar, beispielsweise rotierbar. Das erste Bauteil 70 der Kopplungseinrichtung 60 ist mit dem Rohr 54 starr verbunden, beispielsweise stoff-, kraft- und/oder formschlüssig gefügt oder bereits ursprünglich einstückig gefertigt.

Die verschiedenen vorbestimmten Positionen des zweiten Bauteils 80 relativ zum ersten Bauteil 70 der Kopplungseinrichtung 60 stellen unterschiedliche Konfigurationen der Kopplungseinrichtung 60 dar, die unterschiedliche Funktionen haben. Insbesondere kann bei einer ersten Konfiguration der Kopplungseinrichtung 60 die Glocke 50 zusammen mit dem Rohr 54 und der Kopplungseinrichtung 60 (ggf. über den bereits mit dem distalen Ende des Schafts 30 verbundenen Arbeitseinsatz 40) vom distalen Ende her über den Schaft 30 des Uterus-Manipulators 10 gestülpt werden. Bei einer zweiten Konfiguration der Kopplungseinrichtung 60 kann beispielsweise das Rohr 54 zusammen mit der Kopplungseinrichtung 60 und der Glocke 50 zwischen der in Figur 1 dargestellten Position als distaler Endposition und einer proximalen Endposition relativ zum Schaft 30 verschoben werden, ohne jedoch vom Schaft 30 wieder vollständig getrennt werden zu können. Bei einer dritten Konfiguration der Kopplungseinrichtung 60 kann beispielsweise das Rohr 54 zusammen mit der Glocke 50 und der Kopplungseinrichtung 60 in einer vorbestimmten Position oder in einer von mehreren vorbestimmten Positionen oder in einer beliebigen Position zwischen den vorbestimmten Endpositionen arretiert sein.

Der Handgriff 20 ist um eine Rotationsachse orthogonal zur Längsachse 38 des Schafts 30 und parallel zur Zeichenebene der Figur 1 rotierbar. Die Rotationsachse des Handgriffs 20 ist insbesondere gleichzeitig Rotationsachse des Drehrads 22 zur Arretierung des Handgriffs 20. Der Handgriff 20 ist durch eine oder mehrere Einrichtungen im Inneren des Schafts 30 derart mit dem distalen Ende 33 des Schafts gekoppelt, dass eine Rotation des Handgriffs 20 mit einer Schwenkbewegung des distalen Endes des Schafts 30 und des mit dem distalen Ende starr verbundenen Arbeitseinsatzes 40 um die Schwenkachse des in Figur 1 nicht sichtbaren Gelenks nahe dem distalen Ende des Schafts 30 einhergeht.

Die Glocke 50 und/oder das Gelenk 52 zwischen der Glocke 50 und dem Rohr 54 ist so ausgebildet, dass die Glocke 50 bei ihrer in Figur 1 dargestellten distalen Position formschlüssig mit dem distalen Ende des Schafts 30 gekoppelt ist. Eine durch Rotation des Handgriffs 20 bewirkte Schwenkbewegung des distalen Endes des Schafts 30 und des Arbeitseinsatzes 40 geht deshalb mit einer entsprechenden Schwenkbewegung der Glocke 50 einher. Deshalb weist die Glocke 50 bei der in Figur 1 gezeigten distalen Position des Rohrs 54 und der Kopplungseinrichtung 60 unabhängig von der durch den Handgriff 20 einstellbaren Winkelposition des distalen Endes des Schafts 30 eine vorbestimmte Position relativ zum distalen Ende 33 des Schafts 30 und zum Arbeitseinsatz 40 auf.

Figur 2 zeigt eine weitere schematische axonometrische Darstellung des Uterus-Manipulators aus Figur 1. Die Art der Darstellung entspricht derjenigen der Figur 1.

In Figur 2 ist die Einheit aus Glocke 50, Rohr 54 und Kopplungseinrichtung 60 in einer proximalen Endposition bzw. in einer äußerst proximalen Position relativ zum Schaft 30 dargestellt. In Figur 2 sind das distale Ende 33 des Schafts 30 und das Gelenk 32 nahe dem distalen Ende 33 des Schafts 30 sichtbar.

Figur 3 zeigt eine weitere schematische Darstellung des Uterus-Manipulators aus den Figuren 1 und 2. Im Gegensatz zu den Figuren 1 und 2 zeigt Figur 3 keine axonometrische, sondern eine Seitenansicht. Die Zeichenebene der Figur 3 ist parallel zur Längsachse 38 des Schafts 30 und zur Rotationsachse des Handgriffs 20.

Die Einheit aus Glocke 50, Rohr 54 und Kopplungseinrichtung 60 befindet sich in der auch in Figur 1 gezeigten distalen Position. Die in dieser Situation gemeinsame Schwenkachse des Gelenks 32 nahe dem distalen Ende 33 des Schafts 30 (vgl. Figur 2) und des Gelenks 52 zwischen Glocke 50 und Rohr 54 ist orthogonal zur Zeichenebene der Figur 3.

Figur 4 zeigt eine weitere schematische Darstellung des Uterus-Manipulators 10 aus den Figuren 1 bis 3. Die Art der Darstellung entspricht derjenigen der Figur 3.

Bei der in Figur 4 gezeigten Situation befindet sich die Einheit aus Glocke 50, Rohr 54 und Kopplungseinrichtung 60 in der auch in Figur 2 gezeigten proximalen Endposition.

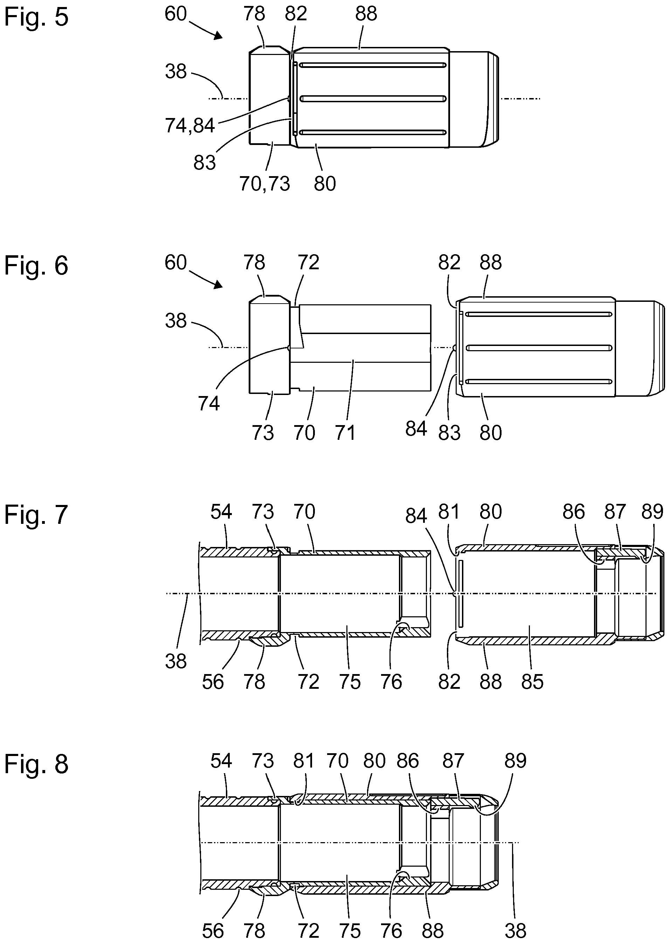

Figur 5 zeigt eine schematische Darstellung einer Ausführungsform der Kopplungseinrichtung 60 des anhand der Figuren 1 bis 4 dargestellten Uterus-Manipulators. Die Kopplungseinrichtung 60 ist im Vergleich zu den Figuren 1 bis 4 vergrößert gezeigt, im Übrigen entspricht die Art der Darstellung derjenigen der Figuren 3 und 4.

Bei der in Figur 5 gezeigten Kopplungseinrichtung 60 ist das zweite Bauteil 80 relativ zum ersten Bauteil 70 um eine Rotationsachse, die beim zusammengesetzten Uterus-Manipulator der Längsachse 38 des Schafts 30 (vgl. Figuren 1 bis 4) entspricht, rotierbar. Die verschiedenen Konfigurationen der Kopplungseinrichtung 60 entsprechen unterschiedlichen Winkelpositionen des zweiten Bauteils 80 relativ zum ersten Bauteil 70 der Kopplungseinrichtung 60.

Figur 6 zeigt eine weitere schematische Darstellung der Kopplungseinrichtung aus Figur 5. Die Art der Darstellung entspricht derjenigen der Figur 5.

In Figur 6 sind das erste Bauteil 70 und das zweite Bauteil 80 der Kopplungseinrichtung 60 voneinander getrennt dargestellt. Sowohl das erste Bauteil 70 als auch das zweite Bauteil 80 sind jeweils im Wesentlichen rohrförmig bzw. weisen im Wesentlichen die Gestalt von Mantelflächen von Kreiszylindern auf. Dabei weist das erste Bauteil 70 großteils einen äußeren Durchmesser auf, der etwas kleiner ist als der innere Durchmesser des zweiten Bauteils 80, um eine spiel- und reibungsarme Führung des ersten Bauteils 70 im zweiten Bauteil 80 zu ermöglichen.

Abweichend von einer ideal zylindermantelförmigen Gestalt weist das erste Bauteil 70 eine sich in Längsrichtung des ersten Bauteils 70 erstreckende Abflachung 71 und eine sich in Richtung des Umfangs des ersten Bauteils 70 erstreckende Nut 72 auf. Die Abflachung 71 verläuft insbesondere gerade und parallel zur Rotationsachse 38. Im Bereich der Abflachung 71 des ersten Bauteils 70 ist die äußere Kontur des Querschnitts des ersten Bauteils 70 nicht kreisbogenförmig, sondern gerade. Die Nut 72 umschließt das erste Bauteil 70 nicht vollständig, sondern bei dem dargestellten Beispiel nur zu drei Vierteln. Die Tiefe der Nut 72 entspricht der Tiefe der Abflachung 71.

Ferner weist das erste Bauteil 70 abweichend von einer ideal kreiszylindermantelförmigen Gestalt an seinem distalen, dem Rohr 54 (vgl. Figuren 1 bis 4) zuzuwendenden Rand einen nach radial außen ragenden Kragen 73 auf. Am proximalen, dem zweiten Bauteil 80 zuzuwendenden Rand des Kragens 73 ist eine Ausnehmung 74 vorgesehen. Ferner weist der Kragen 73 eine Markierung 78 in Gestalt eines sich in axialer Richtung erstreckenden Stegs auf.

Das zweite Bauteil 80 weist an seinem distalen, dem Kragen 73 des ersten Bauteils 70 zuzuwendenden Rand 82 einen elastischen streifenförmigen Bereich 83 mit einer in axialer Richtung nach distal vorstehenden erste Rastnase 84 auf. Der elastische streifenförmige Bereich 83 ist insbesondere durch einen geraden Schnitt bzw. ein schmales Fenster vom übrigen zweiten Bauteil getrennt, so dass beide Enden des elastischen streifenförmigen Bereichs mit dem übrigen zweiten Bauteil 80 starr verbunden sind. Die erste Rastnase 84 ist in der Mitte des elastischen streifenförmigen Bereichs 83 angeordnet. Die Gestalt der ersten Rastnase 84 entspricht der Gestalt der Ausnehmung 74 am Kragen 73 am ersten Bauteil 70.

Ferner weist das zweite Bauteil 80 eine Markierung 88 in Gestalt eines sich in axialer Richtung erstreckenden Stegs auf.

In Figur 5 ist erkennbar, wie die erste Rastnase 84 am elastischen streifenförmigen Bereich 83 in die Ausnehmung 74 am Kragen 73 des ersten Bauteils 70 eingreift und so formschlüssig eine vorbestimmte Winkelposition des zweiten Bauteils 80 relativ zum ersten Bauteil 70 definiert. Am Kragen 73 am ersten Bauteil 70 sind mehrere, insbesondere drei Ausnehmungen 74 vorgesehen, die in Winkelabständen von 90 Grad angeordnet sind. Damit sind drei vorbestimmte Winkelpositionen des zweiten Bauteils 80 relativ zum ersten Bauteil 70 formschlüssig definiert.

Figur 7 zeigt eine schematische Darstellung eines Schnitts durch die Kopplungseinrichtung 60 aus den Figuren 5 und 6. Die Schnittebene der Figur 7 ist parallel zu den Zeichenebenen der Figuren 5 und 6 und enthält die Rotationsachse bzw. die Längsachse 38 des in die Kopplungseinrichtung 60 einzuführenden Schafts 30. Die in Figur 7 gezeigte Blickrichtung auf die Schnittebene ist jedoch entgegengesetzt zur in den Figuren 5 und 6 dargestellten Blickrichtung, um den elastischen streifenförmigen Bereich 83 am zweiten Bauteil 80 sichtbar zu machen. Entsprechend der umgekehrten Blickrichtung sind die Markierung 78 am ersten Bauteil 70 und die Markierung 88 am zweiten Bauteil 80 bei der Darstellung in Figur 7 unten angeordnet.

Die Darstellung in Figur 7 unterscheidet sich von den Darstellungen in den Figuren 5 und 6 ferner dadurch, dass das proximale Ende des Rohrs 54 mit der Riffelung 56 gezeigt ist. Der proximale Rand des Rohrs 54 ist insbesondere durch eine Schweißung, Lötung, Klebung oder auf andere stoff-, kraft- und/oder formschlüssige Weise mit dem distalen Rand des ersten Bauteils 70 gefügt. Zur Verbesserung der Robustheit der Verbindung des Rohrs 54 und des ersten Bauteils 70 der Kopplungseinrichtung 60 überlappen der proximale Rand des Rohrs 54 und der distale Rand des ersten Bauteils 70.

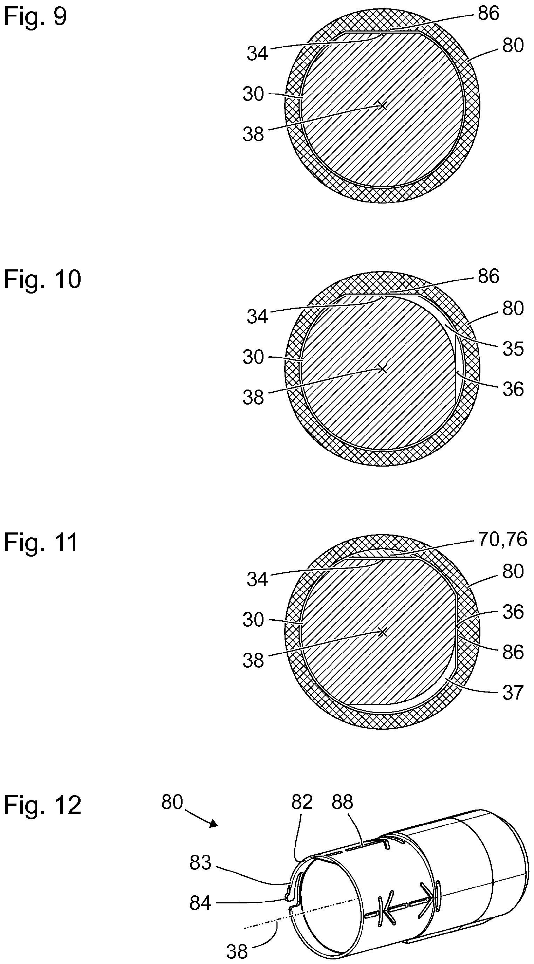

Das erste Bauteil 70 umschließt einen im Wesentlichen kreiszylindrischen Innenraum 75, in den ein nach innen ragender Bereich 76 des ersten Bauteils 70 hineinragt. Die Funktion des nach innen ragenden Bereichs 76 des ersten Bauteils 70 ist anhand der Figuren 9 bis 11 erklärt.

Das zweite Bauteil 80 umschließt einen im Wesentlichen kreiszylindrischen Innenraum 85, dessen Querschnitt weitgehend dem äußeren Querschnitt des ersten Bauteils 75 im Bereich proximal des Kragens 73 entspricht. Das zweite Bauteil 80 weist einen nach innen ragenden Bereich 81 auf. Der Bereich 81 weist im in Figur 7 dargestellten Längsschnitt eine zur Nut 72 am ersten Bauteil 70 korrespondierende Gestalt auf. Im Querschnitt bzw. im Schnitt entlang einer Ebene orthogonal zur Längsachse 38 eines in die Kopplungseinrichtung 60 einzusetzenden Schafts 30 (vgl. Figuren 1 bis 4) weist der Bereich 81 eine zur Abflachung 71 am ersten Bauteil 70 korrespondierende Gestalt auf.

Der in den Innenraum 85 des zweiten Bauteils 80 ragende Bereich 81 des zweiten Bauteils 80 einerseits und die Abflachung 71 (vgl. Figur 6) und die Nut 72 am ersten Bauteil 70 andererseits bilden eine Renkverbindung zur formschlüssigen und lösbaren mechanischen Verbindung der Bauteile 70, 80 der Kopplungseinrichtung 60. Bei geeigneter Ausrichtung des zweiten Bauteils 80 zum ersten Bauteil 70 kann das zweite Bauteil 80 durch eine gerade translatorische Bewegung an das erste Bauteil 70 angesetzt bzw. über dieses gestülpt werden. Bei dieser geraden translatorischen Bewegung greift der in den Innenraum 85 des zweiten Bauteils 80 ragende Bereich 81 in die Abflachung 71 am ersten Bauteil 70 ein. Durch eine nachfolgende rotatorische Bewegung des zweiten Bauteils 80 relativ zum ersten Bauteil 70 können der in den Innenraum 85 ragende Bereich 81 am zweiten Bauteil 80 in die Nut 72 am ersten Bauteil 70 eingeführt und das zweite Bauteil 80 in eine der durch die erste Rastnase 84 und die Ausnehmungen 74 definierten Winkelpositionen gebracht werden, in denen die Bauteile 70, 80 der Kopplungseinrichtung 60 miteinander mechanisch verbunden sind.

Das zweite Bauteil 80 weist ferner einen Bereich 86 auf, der in den Innenraum 85 des zweiten Bauteils 80 ragt. Der Bereich 86 ragt weiter in den Innenraum 85 als der Bereich 81.

Das zweite Bauteil 80 weist ferner einen Stift 87 auf, der sich in Richtung parallel zur Rotationsachse 38 erstreckt und an seinem distalen Ende mit dem nach innen ragenden Bereich 86 des zweiten Bauteils 86 mechanisch starr verbunden ist. Am freien Ende des Stifts 87 ist eine zweite Rastnase 89 vorgesehen. Der Stift 87 kann elastisch ausgebildet sein.

Die zweite Rastnase 89 am zweiten Bauteil 80 kann in die Ausnehmung 39 (vgl. Figur 1) einrasten, um das Erreichen der in den Figuren 2 und 4 gezeigten proximalen Endposition spürbar oder hörbar zu machen und die Kopplungseinrichtung 60 und mit ihr das Rohr 54 und die Glocke 50 in dieser Endposition lösbar zu halten. Entsprechende, in Figur 1 nicht sichtbare weitere Ausnehmungen können an weiteren Stellen am Schaft 30 vorgesehen sein, um das Erreichen entsprechender Positionen spürbar oder hörbar zu machen und die Kopplungseinrichtung 60 mit dem Rohr 54 und der Glocke 50 in diesen Positionen zu halten.

Figur 8 zeigt eine weitere schematische Darstellung eines Schnitts durch die Kopplungseinrichtung 60 aus den Figuren 5 bis 7. Die Art der Darstellung der Figur 8, insbesondere die Schnittebene, entspricht derjenigen der Figur 7. Die Bauteile 70 und 80 der Kopplungseinrichtung 60 sind jedoch nicht wie bei den in den Figuren 6 und 7 gezeigten Situationen voneinander getrennt. Vielmehr zeigt Figur 8 die in Figur 5 gezeigte Situation, bei der die Bauteile 70, 80 der Kopplungseinrichtung 60 miteinander verbunden sind.

Der Bereich 81 am zweiten Bauteil 80 greift in die Nut 72 am ersten Bauteil ein und hält so formschlüssig die Bauteile 70, 80 zusammen. Der nach innen ragende Bereich 76 am ersten Bauteil 70, der nach innen ragende Bereich 86 am zweiten Bauteil 80 und die zweite Rastnase 89 am zweiten Bauteil 80 ragen in den durch den Innenraum des Rohrs 54 und den Innenraum 75 des zweiten Bauteils 70 definierten kreiszylindrischen Raumbereich hinein. Die Funktion der nach innen ragenden Bereiche 76, 86 an den Bauteilen 70, 80 sind in Bezug zu den Figuren 9 bis 11 beschrieben.

Figur 9 zeigt eine schematische Darstellung eines Schnitts durch den anhand der Figuren 1 bis 4 dargestellten Uterus-Manipulator mit der anhand der Figuren 5 bis 9 dargestellten Kopplungseinrichtung. In Figur 9 ist ein Schnitt durch den Uterus-Manipulator in einer Situation gezeigt, bei der die Einheit aus Glocke 50, Rohr 54 und Kopplungseinrichtung 60 relativ zum Schaft 30 weiter distal angeordnet ist als in den Figuren 1 und 3 dargestellt. Die Schnittebene der Figur 9 liegt nahe dem proximalen Rand des zweiten Bauteils 80 und schneidet dessen nach innen ragenden Bereich 86. Das Innere des Schafts 30, in dem insbesondere Einrichtungen zur Kopplung einer Rotationsbewegung des Handgriffs 20 mit einer Schwenkbewegung des distalen Endes 33 des Schafts 30, des Arbeitseinsatzes 40 und der Glocke 50 (vgl. Figuren 1 bis 4) angeordnet sind, ist in Figur 9 nicht gezeigt.

Der Schaft 30 weist die beschriebene erste sich in Richtung parallel zur Längsachse 38 des Schafts 30 erstreckende Abflachung 34 auf, in dessen Bereich die äußere Kontur des Schafts 30 nicht kreisbogenförmig, sondern gerade ist. Die Gestalt des nach innen ragenden Bereichs 86 am zweiten Bauteil 80 korrespondiert zur Abflachung 34 am Schaft 30. Dadurch ist die Winkelposition des zweiten Bauteils 80 relativ zum Schaft 30 festgelegt.

Figur 10 zeigt eine schematische Darstellung eines Schnitts durch den anhand der Figuren 1 bis 4 dargestellten Uterus-Manipulator 10 mit der anhand der Figuren 5 bis 8 dargestellten Kopplungseinrichtung 60. Die Art der Darstellung entspricht derjenigen der Figur 9. In Figur 10 ist ein Schnitt durch den Uterus-Manipulator in der in den Figuren 2 und 4 gezeigten Situation gezeigt, bei der die Einheit aus Glocke 50, Rohr 54 und Kopplungseinrichtung 60 relativ zum Schaft 30 sich in der proximalen Endposition befindet. Die Schnittebene der Figur 10 liegt nahe dem proximalen Rand des zweiten Bauteils 80, schneidet dessen nach innen ragenden Bereich 86 und liegt in der ersten Nut 35 am Schaft 30, die sich in Richtung des Umfangs des Schafts 30 erstreckt.

Die Nut 35 verbindet in Richtung parallel zum Umfang des Schafts 30 die erste Abflachung 34 mit einer zweiten Abflachung 36, die sich parallel zur ersten Abflachung 34 und zur Längsachse 38 des Schafts 30 erstreckt. Der nach innen ragende Bereich 86 am zweiten Bauteil 80 und die erste Nut 35 sind so aufeinander abgestimmt, dass der nach innen ragende Bereich 86 in der ersten Nut 35 bewegt werden kann. Deshalb kann das zweite Bauteil 80 ausgehend von der in Figur 10 gezeigten Winkelposition im Uhrzeigersinn um circa 90 Grad gedreht werden.

Figur 11 zeigt eine schematische Darstellung eines Schnitts durch den anhand der Figuren 1 bis 4 dargestellten Uterus-Manipulator 10 mit der anhand der Figuren 5 bis 9 dargestellten Kopplungseinrichtung 60. Die Art der Darstellung entspricht derjenigen der Figuren 9 und 10. In Figur 11 ist ein Schnitt durch den Uterus-Manipulator in der in den Figuren 1 und 3 gezeigten Situation gezeigt, bei der die Einheit aus Glocke 50, Rohr 54 und Kopplungseinrichtung 60 relativ zum Schaft 30 sich in der distalen Endposition befindet. Die Schnittebene der Figur 10 liegt nahe dem proximalen Rand des zweiten Bauteils 80, schneidet dessen nach innen ragenden Bereich 86 und liegt in einer zweiten Nut 37 am Schaft 30, die sich in Richtung des Umfangs des Schafts 30 erstreckt.

Die Nut 37 erstreckt sich von der zweiten Abflachung 36 ausgehend in einer Richtung weg von der ersten Abflachung 34 über einen Winkel von circa 90 Grad. Der nach innen ragende Bereich 86 am zweiten Bauteil 80 und die zweite Nut 37 sind so aufeinander abgestimmt, dass der nach innen ragende Bereich 86 in der zweiten Nut 37 bewegt werden kann. Deshalb kann das zweite Bauteil 80 ausgehend von der in Figur 11 gezeigten Position im Uhrzeigersinn um einen Winkel von circa 90 Grad gedreht werden.

In Figur 11 ist der nach innen ragende Bereich 76 des ersten Bauteils 70 sichtbar, der in die erste Abflachung 34 am Schaft 30 eingreift. Der Formschluss zwischen dem nach innen ragenden Bereich 76 des ersten Bauteils 70 und der Abflachung 34 am Schaft 30 bewirkt, dass das erste Bauteil 70 und mit ihm das Rohr 54 und die Glocke 50 (vgl. Figuren 1 bis 4) nicht um die Längsachse 38 des Schafts 30 rotiert werden können.

Figur 12 zeigt eine schematische axonometrische Darstellung eines zweiten Bauteils 80 einer weiteren Ausführungsform einer Kopplungseinrichtung 60 des anhand der Figuren 1 bis 4 dargestellten Uterus-Manipulators 10. Das in Figur 8 gezeigte zweite Bauteil 80 ähnelt in einigen Merkmalen, Eigenschaften und Funktionen dem zweiten Bauteil der anhand der Figuren 5 bis 11 dargestellten Kopplungseinrichtung. Das in Figur 12 gezeigte zweite Bauteil 80 ist insbesondere dafür vorgesehen, mit dem anhand der Figuren 5 bis 11 dargestellten ersten Bauteil 70 oder mit einem ähnlichen Bauteil zu einer Kopplungseinrichtung kombiniert zu werden. Nachfolgend sind insbesondere Merkmale, Eigenschaften und Funktionen beschrieben, in denen das in Figur 12 gezeigte zweite Bauteil 80 sich von dem anhand der Figuren 5 bis 11 dargestellten zweiten Bauteil unterscheidet.

Das in Figur 12 gezeigte zweite Bauteil 80 unterscheidet sich von dem anhand der Figuren 5 bis 11 dargestellten zweiten Bauteil insbesondere dadurch, dass die erste Rastnase 84 am freien Ende eines nur einseitig eingespannten bzw. nur am gegenüberliegenden Ende starr mit dem übrigen zweiten Bauteil 80 verbundenen elastischen streifenförmigen Bereich 83 angeordnet ist. Der elastische streifenförmige Bereich 83 wird anders als bei der anhand der Figuren 5 bis 11 dargestellten Ausführungsform nicht durch einen geraden, sondern durch einen L-förmigen Schnitt am distalen, dem Kragen 73 am ersten Bauteil 70 (vgl. Figuren 5 bis 8) zuzuwendenden Rand 82 gebildet.

Bei der in Figur 12 gezeigten Kopplungseinrichtung sind Markierungen 88 für die vorbestimmten Winkelpositionen nicht in Gestalt eines Stegs, sondern in Gestalt von in die äußere Oberfläche des zweiten Bauteils 80 eingravierten (laserbeschrifteten) Symbolen vorgesehen.

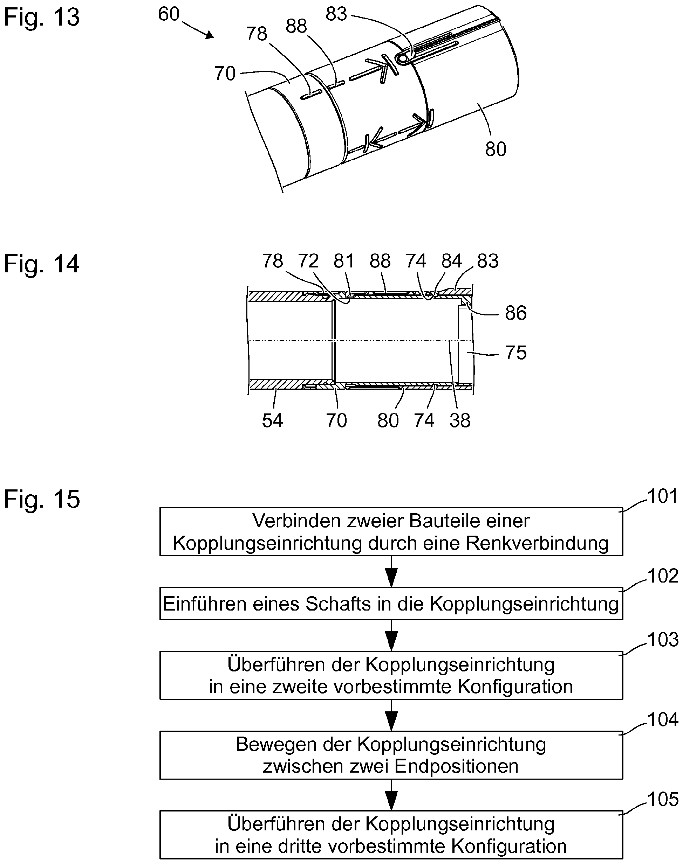

Figur 13 zeigt eine schematische axonometrische Darstellung einer weiteren Ausführungsform einer Kopplungseinrichtung des anhand der Figuren 1 bis 4 dargestellten Uterus-Manipulators 10, die in einigen Merkmalen, Eigenschaften und Funktionen den anhand der Figuren 5 bis 12 dargestellten Kopplungseinrichtungen ähnelt. Nachfolgend sind insbesondere Merkmale, Eigenschaften und Funktionen beschrieben, in denen die in Figur 13 gezeigte Kopplungseinrichtung 60 sich von den anhand der Figuren 5 bis 12 dargestellten Kopplungseinrichtungen unterscheidet.

Bei der in Figur 13 gezeigten Kopplungseinrichtung ist der elastische streifenförmige Bereich 83 nicht am distalen Rand des zweiten Bauteils 80 angeordnet und nicht parallel zum Umfang, sondern parallel zur Längsachse orientiert. Ein Ende des elastischen streifenförmigen Bereichs 83 ist starr mit dem übrigen zweiten Bauteil 80 verbunden. Am anderen, gegenüberliegenden Ende ist eine in Figur 13 nicht sichtbare Rastnase vorgesehen, die nach radial innen in eine in Figur 13 ebenfalls nicht sichtbare Ausnehmung im ersten Bauteil 70 eingreift. Der elastische streifenförmige Bereich 83 ist anders als bei den anhand der Figuren 5 bis 12 dargestellten Ausführungsformen nicht durch einen geraden oder einen L-förmigen Schnitt, sondern durch einen U-förmigen Schnitt gebildet.

Figur 14 zeigt eine schematische Darstellung eines Schnitts durch die anhand der Figur 13 dargestellte Kopplungseinrichtung 60. Die Schnittebene enthält die Längsachse 38 eines in die Kopplungseinrichtung 60 einzuführenden Schafts 30 (vgl. Figuren 1 bis 4 und 9 bis 11), um die das zweite Bauteil 80 relativ zum ersten Bauteil 70 rotierbar ist.

Bei der in Figur 14 gezeigten Situation greift die erste Rastnase 84 am freien Ende des elastischen streifenförmigen Bereichs 83 in eine von mehreren korrespondierenden Ausnehmungen 74 an der äußeren Oberfläche des ersten Bauteils 70 ein. Wenn das zweite Bauteil 80 aus der in den Figuren 13 und 14 gezeigten vorbestimmten Position heraus rotiert wird, wird die erste Rastnase 84 gegen die elastische Kraft des elastischen streifenförmigen Bereichs 83 nicht wie bei den anhand der Figuren 5 bis 12 dargestellten Ausführungsformen in axialer Richtung bzw. in Richtung parallel zur Rotationsachse 38, sondern in radialer Richtung bzw. in Richtung orthogonal zur Rotationsachse 38 ausgelenkt.

Figur 15 zeigt ein schematisches Flussdiagramm eines Verfahrens zur Vorbereitung eines Uterus-Manipulators oder eines anderen medizinischen Instruments für seine Verwendung bei einer medizinischen Maßnahme. Obwohl das Verfahren auch mit medizinischen Instrumenten durchführbar ist, die Merkmale, Eigenschaften und Funktionen aufweisen, die von den anhand der Figuren 1 bis 14 dargestellten abweichen, sind nachfolgend Bezugszeichen aus den Figuren 1 bis 14 verwendet, um das Verständnis zu vereinfachen.

Bei einem optionalen ersten Schritt 101 werden zwei Bauteile 70, 80 einer Kopplungseinrichtung 60 durch eine Renkverbindung miteinander verbunden. Der erste Schritt 101 wird insbesondere nach einer vorangehenden Verwendung und einer darauf folgenden Reinigung der Kopplungseinrichtung ausgeführt. Die Kopplungseinrichtung ist insbesondere ständig oder zum Zeitpunkt der Ausführung des ersten Schritts 101 Teil eines Bestandteils 50, 54, 60 eines medizinischen Instruments 10, insbesondere eines Uterus-Manipulators. Insbesondere ist das erste Bauteil 70 der Kopplungseinrichtung 60 mit dem Bestandteil 50, 54 oder einem Teil 54 davon ständig und starr verbunden. Dabei wird insbesondere eine erste Konfiguration der Kopplungseinrichtung 60 hergestellt, bei der das zweite Bauteil 80 eine erste vorbestimmte Winkelposition relativ zum ersten Bauteil 70 der Kopplungseinrichtung 60 einnimmt.

Bei einem zweiten Schritt 102 wird ein Schaft 30 als Teil eines zweiten Bestandteils 20, 30, 40 des medizinischen Instruments 10 in die Kopplungseinrichtung 60 in ihrer ersten vorbestimmten Konfiguration eingeführt. Dabei wird die Kopplungseinrichtung 60 in ihrer ersten vorbestimmten Konfiguration insbesondere vom distalen Ende 33 her über den Schaft 30 gestülpt.

Bei einem dritten Schritt 103 wird die Kopplungseinrichtung 60 in eine zweite Konfiguration überführt, um die Kopplungseinrichtung 60 mit dem Schaft 30 zu verbinden. Dazu wird insbesondere das zweite Bauteil 80 der Kopplungseinrichtung in eine der zweiten vorbestimmten Konfiguration entsprechende zweite vorbestimmte Winkelposition relativ zum ersten Bauteil 70 der Kopplungseinrichtung 60 gebracht. Dadurch ist die Kopplungseinrichtung 60 insbesondere derart mit dem Schaft 30 mechanisch verbunden, dass die Kopplungseinrichtung 60 relativ zum Schaft und damit der Bestandteil 20, 30, 40 und der Bestandteil 50, 54, 60 des medizinischen Instruments 10 innerhalb eines vorbestimmten Bereichs relativ zueinander bewegt, jedoch nicht mehr voneinander getrennt werden können.

Bei einem optionalen vierten Schritt 104 kann die Kopplungseinrichtung 60 zwischen zwei Endpositionen hin- und herbewegt werden, insbesondere entlang eines geraden linearen Pfads. Damit können auch der Bestandteil 20, 30, 40 und der Bestandteil 50, 54, 60 des medizinischen Instruments 10 relativ zueinander zwischen den vorbestimmten Endpositionen bewegt werden.

Bei einem optionalen fünften Schritt 105 wird die Kopplungseinrichtung 60 in eine dritte vorbestimmte Konfiguration überführt, insbesondere durch Rotation des zweiten Bauteils 80 in eine der dritten vorbestimmten Konfiguration entsprechende dritte vorbestimmte Winkelposition des zweiten Bauteils 80 relativ zum ersten Bauteil 70 der Kopplungseinrichtung 60. Bei der dritten vorbestimmten Konfiguration ist die Kopplungseinrichtung 60 am Schaft 30 in einer vorbestimmten Position oder in einer von mehreren vorbestimmten Positionen oder in einer beliebigen Position zwischen den beiden Endpositionen arretiert.

### Bezugszeichen

- 10: Uterus-Manipulator
- 12: proximales Ende des Uterus-Manipulators 10
- 14: distales Ende des Uterus-Manipulators 10
- 20: Handgriff am proximalen Ende 12 des Uterus-Manipulators 10
- 22: Drehrad zur Arretierung des Handgriffs 20
- 30: Schaft des Uterus-Manipulators 10
- 31: proximales Ende des Schafts 30
- 32: Gelenk nahe dem distalen Ende 33 des Schafts 30
- 33: distales Ende des Schafts 30
- 34: erste, sich in Längsrichtung des Schafts 30 erstreckende Abflachung am Schaft 30
- 35: erste, sich in Richtung des Umfangs erstreckende Nut am proximalen Ende des Schafts 30
- 36: zweite, sich in Längsrichtung des Schafts 30 erstreckende Abflachung am Schaft 30
- 37: zweite, sich in Richtung des Umfangs erstreckende Nut am distalen Ende des Schafts 30
- 38: Längsachse des Schafts 30
- 39: Ausnehmung nahe dem proximalen Ende 31 des Schafts 30
- 40: Arbeitseinsatz am distalem Ende 33 des Schafts 30
- 42: Gewinde am Arbeitseinsatz 40
- 50: Glocke
- 52: Gelenk zwischen der Glocke 50 und dem Rohr 54
- 54: Rohr
- 56: Riffelung am Rohr 54
- 60: Kopplungseinrichtung zur mechanischen Verbindung des Rohrs 54 mit dem Schaft 30
- 70: erstes Bauteil der Kopplungseinrichtung 60
- 71: sich in Längsrichtung des ersten Bauteils 70 erstreckende Abflachung an äußerer Oberfläche des ersten Bauteils 70
- 72: sich in Richtung des Umfangs erstreckende Nut an äußerer Oberfläche des ersten Bauteils 70
- 73: nach radial außen ragender Kragen am ersten Bauteil 70
- 74: Nut oder Ausnehmung am Kragen 73
- 75: im Wesentlichen kreiszylindrischer Innenraum des ersten Bauteils 70
- 76: in den Innenraum 75 ragender Bereich des ersten Bauteils 70
- 78: Markierung am ersten Bauteil 70
- 80: zweites Bauteil der Kopplungseinrichtung 60
- 81: zur Abflachung 81 am ersten Bauteil 70 korrespondierender, nach innen ragender Bereich des zweiten Bauteils 80
- 82: distaler Rand des zweiten Bauteils 80
- 83: elastischer streifenförmiger Bereich am zweiten Bauteil 80
- 84: in axialer Richtung vorstehende erste Rastnase am elastischen streifenförmigen Bereich 83
- 85: im Wesentlichen kreiszylindrischer Innenraum des zweiten Bauteils 80
- 86: in den Innenraum 85 ragender Bereich des zweiten Bauteils 80
- 87: Stift am nach innen ragenden Bereich 86 am zweiten Bauteil 80
- 88: Markierung am zweiten Bauteil 80
- 89: zweite Rastnase am Stift 87

- 101: erster Schritt (Verbinden zweier Bauteile einer Kopplungseinrichtung durch eine Renkverbindung)
- 102: zweiter Schritt (Einführen eines Schafts in die Kopplungseinrichtung)
- 103: dritter Schritt (Überführen der Kopplungseinrichtung in eine zweite vorbestimmte Konfiguration)
- 104: vierter Schritt (Bewegen der Kopplungseinrichtung zwischen zwei Endpositionen)
- 105: fünfter Schritt (Überführen der Kopplungseinrichtung in eine dritte vorbestimmte Konfiguration)

## Patentansprüche

1. **Kopplungseinrichtung** (60) zur mechanischen Verbindung eines ersten Bestandteils (30) und eines zweiten Bestandteils (50) eines medizinischen Instruments (10), mit:
einem **ersten Bauteil** (70);
einem **zweiten Bauteil** (80), wobei das erste Bauteil (70) und das zweite Bauteil (80) ausgebildet sind, um durch eine Renkverbindung, die im verbundenen Zustand eine Rotation des zweiten Bauteils (80) relativ zum ersten Bauteil (70) um eine Rotationsachse (38) ermöglicht, miteinander verbunden zu werden;
einer **Rasteinrichtung** (74, 83, 84) zum alternativen Arretieren des zweiten Bauteils (80) in einer ersten vorbestimmten Winkelpositionen oder in einer zweiten vorbestimmten Winkelposition relativ zum ersten Bauteil (70),
wobei die Rasteinrichtung eine **Rastnase** (84) und **mehrere** zur Rastnase (84) korrespondierende **Ausnehmungen** (74), die die erste vorbestimmte Winkelposition und die zweite vorbestimmte Winkelposition vorbestimmen, oder eine **Ausnehmung** und **mehrere** zur Ausnehmung korrespondierende **Rastnasen,** die die mehreren verschiedenen Winkelpositionen vorbestimmen, umfasst, **dadurch gekennzeichnet, dass** zumindest entweder eine **Rastnase** (84) oder eine Ausnehmung (74) der Rasteinrichtung (74, 83, 84) in einer Richtung, die im Wesentlichen **parallel** zur Rotationsachse (38) ist, elastisch **auslenkbar** ist.

2. Kopplungseinrichtung (60) nach Anspruch 1, bei der
die Rasteinrichtung (74, 83, 84) ferner vorgesehen und ausgebildet ist, um das zweite Bauteil (80) relativ zum ersten Bauteil (70) alternativ in einer dritten vorbestimmten Winkelposition zu arretieren,
die Kopplungseinrichtung (60) vorgesehen und ausgebildet ist, um bei der **dritten Winkelposition** eine im Wesentlichen **starre Verbindung** des ersten Bestandteils (30) und des zweiten Bestandteils (50) des medizinischen Instruments (10) zu schaffen.

3. Kopplungseinrichtung (60) nach dem vorangehenden Anspruch, bei der zumindest entweder eine **Rastnase** (84) oder eine Ausnehmung (74) der Rasteinrichtung (74, 83, 84) in einer Richtung, die im Wesentlichen **orthogonal** zur Rotationsachse (38) ist, elastisch auslenkbar ist.

4. Kopplungseinrichtung (60) nach dem vorangehenden Anspruch, bei der eine Rastnase (84) der Rasteinrichtung (74, 83, 84) an einer einseitig oder **beidseitig befestigten streifenförmigen elastischen Einrichtung** (83) angeordnet ist.

5. Kopplungseinrichtung (60) nach einem der vorangehenden Ansprüche, bei der das erste Bauteil (70) und das zweite Bauteil (80) jeweils im Wesentlichen **rohrförmig** sind, und bei der das erste Bauteil (70) zumindest teilweise in dem zweiten Bauteil (80) angeordnet ist, wenn das zweite Bauteil (80) mit dem ersten Bauteil (70) durch die Renkverbindung verbunden ist.

6. Kopplungseinrichtung (60) nach dem vorangehenden Anspruch, bei der
die Kopplungseinrichtung (60) einen im Wesentlichen **kreiszylindrischen Innenraum** (75, 85) umschließt,
das erste Bauteil (70) einen **in den** kreiszylindrischen **Innenraum** (75, 85) **ragenden** Bereich (76) aufweist,
das zweite Bauteil (80) einen **in den** kreiszylindrischen **Innenraum** (75, 85) **ragenden** Bereich (86) aufweist.

7. Kopplungseinrichtung (60) nach dem vorangehenden Anspruch, bei der
das erste Bauteil (70) einen **Kragen** (73) aufweist,
die Rasteinrichtung (74, 83, 84) an dem Kragen (73) am ersten Bauteil (70) und an einem dem Kragen (73) gegenüberliegenden Rand (82) des zweiten Bauteils (80) angeordnet ist.

8. **Medizinisches Instrument** (10), mit:
einem **Schaft** (30);
einer **Wirkeinrichtung** (50) zur Einwirkung auf einen Teil eines menschlichen oder tierischen Körpers;
einer **Kopplungseinrichtung** (60) nach einem der vorangehenden Ansprüche mit mehreren vorbestimmten Konfigurationen, zur mechanischen Verbindung der Wirkeinrichtung (50) mit dem Schaft (30);
wobei bei einer **ersten Konfiguration** der Kopplungseinrichtung (60) ein **Zusammensetzen** und eine **zerstörungsfreie Trennung** von Wirkeinrichtung (50) und Schaft (30) möglich sind,
wobei bei einer **zweiten Konfiguration** der Kopplungseinrichtung (60) die Wirkeinrichtung (50) mit dem Schaft (30) derart mechanisch verbunden ist, dass die Wirkeinrichtung (50) entlang dem Schaft (30) zwischen einer proximalen Position und einer distalen Position **verschiebbar** ist.

9. Medizinisches Instrument (10) nach dem vorangehenden Anspruch, bei dem bei einer **dritten Konfiguration** der Kopplungseinrichtung (60) die Wirkeinrichtung (50) in einer Position am Schaft (30) **arretiert** ist.

10. Medizinisches Instrument (10) nach einem der Ansprüche 8 und 9, bei dem die Kopplungseinrichtung ein erstes Bauteil (70), das mit der Wirkeinrichtung (50) verbunden ist, und ein zweites Bauteil (80), das relativ zu dem ersten Bauteil (70) um die Längsachse (38) des Schafts (30) rotierbar ist, und das bei verschiedenen Konfigurationen der Kopplungseinrichtung (60) **unterschiedliche Winkelpositionen** relativ zum ersten Bauteil (70) einnimmt, umfasst.

## Claims

1. Coupling device (60) for mechanically connecting a first structural part (30) and a second structural part (50) of a medical instrument (10), with:
a first component (70);
a second component (80), wherein the first component (70) and the second component (80) are designed to be connected to each other by a bayonet connection which permits a rotation of the second component (80) relative to the first component (70) about a rotation axis (38) in the connected state;
a detent device (74, 83, 84) for alternatively locking the second component (80) in a first predetermined angle positions or in a second predetermined angle position relative to the first component (70),
wherein the detent device comprises a detent lug (84) and several recesses (74) which correspond to the detent lug (84) and which predetermine the first predetermined angle position and the second predetermined angle position, or a recess and several detent lugs which correspond to the recess and which predetermine the several different angle positions,
**characterized in that** at least either a detent lug (84) or a recess (74) of the detent device (74, 83, 84) is elastically deflectable in a direction that is substantially parallel to the rotation axis (38).

2. Coupling device (60) according to Claim 1, in which
the detent device (74, 83, 84) is moreover provided and designed to lock the second component (80) relative to the first component (70) alternatively in a third predetermined angle position,
the coupling device (60) is provided and designed to create, in the third angle position, a substantially rigid connection of the first structural part (30) and of the second structural part (50) of the medical instrument (10).

3. Coupling device (60) according to the preceding claim, in which at least either a detent lug (84) or a recess (74) of the detent device (74, 83, 84) is elastically deflectable in a direction that is substantially orthogonal to the rotation axis (38) .

4. Coupling device (60) according to the preceding claim, in which a detent lug (84) of the detent device (74, 83, 84) is arranged on a strip-shaped, elastic device (83) secured at one end or at both ends.

5. Coupling device (60) according to one of the preceding claims, in which the first component (70) and the second component (80) are each substantially tubular, and in which the first component (70) is arranged at least partially in the second component (80) when the second component (80) is connected to the first component (70) by the bayonet connection.

6. Coupling device (60) according to the preceding claim, in which
the coupling device (60) encloses a substantially circular cylindrical interior (75, 85),
the first component (70) has a region (76) protruding into the circular cylindrical interior (75, 85),
the second component (80) has a region (86) protruding into the circular cylindrical interior (75, 85).

7. Coupling device (60) according to the preceding claim, in which
the first component (70) has a collar (73),
the detent device (74, 83, 84) is arranged on the collar (73) on the first component (70) and on an edge (82) of the second component (80) lying opposite the collar (73).

8. Medical instrument (10), with:
a shaft (30) ;
an effecting device (50) for acting on part of a human or animal body;
a coupling device (60) according to one of the preceding claims, with several predetermined configurations, for mechanically connecting the effecting device (50) to the shaft (30);
wherein a first configuration of the coupling device (60) permits a fitting together and a nondestructive separation of effecting device (50) and shaft (30),
wherein, in a second configuration of the coupling device (60), the effecting device (50) is mechanically connected to the shaft (30) in such a way that the effecting device (50) is displaceable along the shaft (30) between a proximal position and a distal position.

9. Medical instrument (10) according to the preceding claim, in which, in a third configuration of the coupling device (60), the effecting device (50) is locked in a position on the shaft (30).

10. Medical instrument (10) according to either of Claims 8 and 9, in which the coupling device comprises a first component (70), which is connected to the effecting device (50), and a second component (80), which is rotatable relative to the first component (70) about the longitudinal axis (38) of the shaft (30) and which, in various configurations of the coupling device (60), adopts different angle positions relative to the first component (70).

## Revendications

1. Dispositif d'accouplement (60) pour la connexion mécanique d'un premier constituant (30) et d'un deuxième constituant (50) d'un instrument médical (10), comprenant :
un premier composant (70) ;
un deuxième composant (80), le premier composant (70) et le deuxième composant (80) étant réalisés de manière à être connectés l'un à l'autre par une connexion à baïonnette qui, dans l'état connecté, permet une rotation du deuxième composant (80) par rapport au premier composant (70) autour d'un axe de rotation (38) ;
un dispositif d'encliquetage (74, 83, 84) pour bloquer en alternance le deuxième composant (80) dans une première positions angulaire prédéterminée ou dans une deuxième position angulaire prédéterminée par rapport au deuxième composant (70),
le dispositif d'encliquetage comprenant un ergot d'encliquetage (84) et plusieurs évidements (74) correspondant à l'ergot d'encliquetage (84), lesquels prédéfinissent la première position angulaire prédéfinie et la deuxième position angulaire prédéfinie, ou un évidement et plusieurs ergots d'encliquetage correspondant à l'évidement, lesquels prédéfinissent les plusieurs positions angulaires différentes,
**caractérisé en ce**
**qu'**au moins soit un ergot d'encliquetage (84) soit un évidement (74) du dispositif d'encliquetage (74, 83, 84) peut être dévié élastiquement dans une direction qui est essentiellement parallèle à l'axe de rotation (38).

2. Dispositif d'accouplement (60) selon la revendication 1, dans lequel
le dispositif d'encliquetage (74, 83, 84) est en outre prévu et réalisé de manière à bloquer le deuxième composant (80) par rapport au premier composant (70) en alternance dans une troisième position angulaire prédéterminée,
le dispositif d'accouplement (60) étant prévu et réalisé pour créer, dans la troisième position angulaire, une connexion essentiellement rigide du premier constituant (30) et du deuxième constituant (50) de l'instrument médical (10).

3. Dispositif d'accouplement (60) selon la revendication précédente, dans lequel au moins soit un ergot d'encliquetage (84) soit un évidement (74) du dispositif d'encliquetage (74, 83, 84) peut être dévié élastiquement dans une direction qui est essentiellement perpendiculaire à l'axe de rotation (38).

4. Dispositif d'accouplement (60) selon la revendication précédente, dans laquelle un ergot d'encliquetage (84) du dispositif d'encliquetage (74, 83, 84) est disposé au niveau d'un dispositif élastique (83) en forme de bandes fixé d'un côté ou des deux côtés.

5. Dispositif d'accouplement (60) selon l'une quelconque des revendications précédentes, dans lequel le premier composant (70) et le deuxième composant (80) sont à chaque fois essentiellement tubulaires, et dans lequel le premier composant (70) est disposé au moins en partie dans le deuxième composant (80), lorsque le deuxième composant (80) est connecté au premier composant (70) par la connexion à baïonnette.

6. Dispositif d'accouplement (60) selon la revendication précédente, dans lequel
le dispositif d'accouplement (60) entoure un espace intérieur (75, 85) essentiellement cylindrique circulaire,
le premier composant (70) présente une région (76) pénétrant dans l'espace intérieur cylindrique circulaire (75, 85),
le deuxième composant (80) présente une région (86) pénétrant dans l'espace intérieur cylindrique circulaire (75, 85).

7. Dispositif d'accouplement (60) selon la revendication précédente, dans lequel le premier composant (70) présente un collet (73),
le dispositif d'encliquetage (74, 83, 84) étant disposé au niveau du collet (73) sur le premier composant (70) et au niveau d'un bord (82) du deuxième composant (80) opposé au collet (73).

8. Instrument médical (10), comprenant :
une tige (30) ;
un dispositif d'actionnement (50) pour agir sur une partie d'un corps humain ou animal ;
un dispositif d'accouplement (60) selon l'une quelconque des revendications précédentes comprenant plusieurs configurations prédéterminées pour la connexion mécanique du dispositif d'actionnement (50) à la tige (30) ;
dans une première configuration du dispositif d'accouplement (60), un assemblage et une séparation sans destruction du dispositif d'actionnement (50) de la tige (30) étant possibles,
dans une deuxième configuration du dispositif d'accouplement (60), le dispositif d'actionnement (50) étant connecté mécaniquement à la tige (30) de telle sorte que le dispositif d'actionnement (50) puisse être déplacé le long de la tige (30) entre une position proximale et une position distale.

9. Instrument médical (10) selon la revendication précédente, dans lequel, dans une troisième configuration du dispositif d'accouplement (60), le dispositif d'actionnement (50) est bloqué dans une position sur la tige (30).

10. Instrument médical (10) selon l'une quelconque des revendications 8 et 9, dans lequel le dispositif d'accouplement comprend un premier composant (70) qui est connecté au dispositif d'actionnement (50), et un deuxième composant (80) qui peut tourner par rapport au premier composant (70) autour de l'axe longitudinal (38) de la tige (30) et qui adopte différentes positions angulaires par rapport au premier composant (70) dans différentes configurations du dispositif d'accouplement (60).
